# EUROPEAN PATENT APPLICATION

(11) **EP 1 584 285 A1**
(43) Date of publication of application: **12.10.2005**
(21) Application number: 04101423.4
(22) Date of filing: 06.04.2004
(51) Int. Cl.: A61B 5/00, G01N 21/64, G01J 1/50

(54) **Calibration aid**

(71) Applicant: Pulsion Medical Systems AG, 81829 München (DE)
(72) Inventor: Pfeiffer, Dr.Ulrich J., 81667 München (DE)
(74) Representative: Kehl, Günther

(57) **Abstract**

The calibration aid according to the application may be assembled as follow. First, the standard reference substance (6) is prepared by dissolving ICG dye and albumin protein in water. Then, the carrier sheet (1) of fleece material is soaked therein and dried. After drying the carrier sheet (1), a thin well defined layer of protein bound dye is present at the surface of the fleece material. The carrier sheet (1) and the backing sheet (2) are laminated into a plastic card. For this, the plastic layers (4a,4b) may be laminated tightly together in the framing region (5) for example by welding or by use of adhesive. The plastic card is then sterilized and packed into a sealed package.

## Description

The present invention refers to a calibration aid for fluorescence measurement applications.

An important field of application of fluorescence measurements is the field of non-invasive tissue perfusion quantification of a patient. A non invasive tissue perfusion imaging and quantification system is described in EP 1 210 906 A2. The patient is monitored with a digital camera while injecting indocyanine green (ICG, a fluorescent dye with an absorption maximum in the near infrared range) and irradiating the tissue of interest using a radiation source emitting radiation in the near infrared range. From the fluorescence signal of the tissue of interest (i.e. the light emitted by the fluorescent dye present in the blood flowing through the tissue) the perfusion state of the tissue can be dermined using appropriate algorithms, such as disclosed in EP 1 210 906 A2. Equipment for intraoperative use of ICG measurements for determining tissue perfusion is further disclosed in DE 10059070 C1.

Advantageously, an external fluorescence standard is used when performing the measurements. It is placed next to the tissue of interest and, when irradiated, emits a constant and defined fluorescence signal which is recorded together with the fluorescence signal of the tissue of interest.

The use of a fluorescence standard allows to directly compare different measurements and to normalize measurement results based on the defined signal intensity of the fluorescence standard. It further allows compensating changes in the measurement conditions during a measurement (such as change in intensity of ambient light, change of exposure parameters of the camera or change of parameters of the radiation).

For reference purposes as described above, pure dry ICG dye is usually dissolved in water or methanol immediately before use. With this liquid sample an in vitro fluorescence measurement can be performed as described above. However, this practice is time consuming. Since ICG is not stable when exposed to air humidity and light, the reference standard must be prepared immediately before use, which is a major disadvantage when used in connection with urgent surgery. Moreover, the absorption and fluorescence properties of such pure dissolved standard samples are not equal to the properties of the protein bound dye after injection to a patient, e.g. the absorption maximum is shifted from 780nm to 805nm.

It is also not possible to use pure dry ICG dye for reference purposes as described above, since pure ICG powder also exhibits absorption and fluorescence properties different from dissolved ICG. Further, dry ICG may alter its properties due to ambient humidity

Therefore, it is an object of the present invention to provide an easy to use ICG reference standard which is long time stable and does not need special preparation before use. Further, it is an object of the present invention to provide a method for producing such a reference standard.

According to one aspect of the present invention, the object is achieved by a calibration aid as defined in claim 1, which can be used as an external reference standard as described above. Preferred embodiments of the calibration aid may be designed as defined in any of the claims 2-14. According to a particularly advantageous embodiment of the present invention, a calibration aid is enclosed in packaging material keeping it sterile before actual use as a disposable. Such a calibration kit, which is also defined in claim 15, may be stored for longer periods of time without imposing on the patient the risk of an infection

A calibration aid, or calibration kit, respectively, of the type described herein may be used together with commercially available angiography systems such as the IC-VIEW (trademark) system by Pulsion Medical Systems AG or similar systems. According to the present invention, a calibration aid as described herein can also be used as a fluorescence or absorption standard for use with other methods using injected indicator dyes.

According to the present invention, a calibration aid of the type mentioned can be manufactured using a method as defined in claim 16. Preferred embodiments of this method may be designed as defined in any of the claims 17-23.

While in most embodiments the porous carrier body is flat and preferably made of textile sheet material (woven or non-woven), the porous carrier body may include sponge-like material, a zeolite bulk, sintered material of various types or other porous materials. Generally, a high volume specific surface area of the carrier body, preferably above 40 000 [1/m], more preferably above 200 000 [1/m], will be advantageous in connection with the present invention, and further the carrier body material has preferably a porosity of above 80%.

In the following, the invention is described, by way of non-limiting example, with reference to the accompanying schematic drawings, which are not to scale, and wherein
- Fig. 1: shows a plan view of the front side of a calibration aid according to the present invention,
- Fig. 2: shows a plan view of the back side of the calibration aid of fig. 1, and
- Fig. 3: shows a cross sectional view of the calibration aid of figs. 1 and 2 with a section plane orthogonal to the drawing plane of figs. 1 and 2, with the section plane being indicated by the broken line A-A' in figs. 1 and 2. The thickness of the individual layers is magnified by a large factor for illustrative purposes.

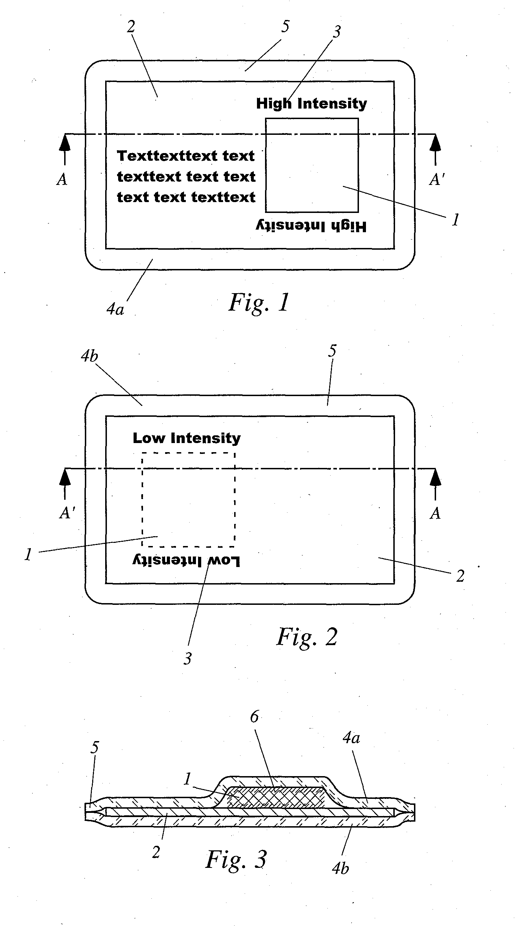

The calibration aid depicted in Figs. 1-3 is designed as an easy to handle card-like object and comprises a fleece carrier sheet 1, a white paper backing sheet 2 with indicia 3 printed thereon, and two transparent plastic layers 4a, 4b, laminated tightly together in the framing region 5. Preferably, the calibration aid is provided as a disposable sterile product, packed as a single device in a Tyvek® covered blister bag or other packaging material capable of preventing the outer surface of the calibration aid from being contaminated.

The fleece carrier sheet 1 may be comprised of polyvinyl-alcohol fibers that are cross-linked with the help of a chemical agent. Fleece products of this type are available, for example, from Freudenberg Hauhaltsprodukte KG. The carrier sheet 1 is laden with a reference standard substance 6 comprising albumin protein and ICG fluorescent dye.

The white paper backing sheet 2 extends beyond the fleece carrier sheet 1. Commercially available paper of 80 grams per square meter is, among others, a suitable material for the backing sheet 2. However, the backing sheet 2 may also be comprised of a plastic material. Preferably, the backing sheet 2 has a certain degree of transparency for both electro-magnetic radiation in the spectral range exciting the reference standard substance 6 to fluoresce and the light emitted due to fluorescence. Thus, the calibration aid can be used as a high intensity standard when irradiated and viewed from the front side and as a low intensity standard when irradiated and viewed from the back side: By turning the plastic card, the backing sheet 2 will attenuate the optical intensity, providing a second reference intensity level.

The indicia 3 may include instruction notes and other identification means. Therefore, the used reference standard may easily be documented by the camera also applied for fluorescence measurements.

PVC film, such as commercially available from Klöckner Pentaplast (e.g. Pentafood LM 176), is a material suitable for the transparent plastic layers 4a, 4b, but other materials capable of acting as a barrier against water diffusion due to ambient humidity may also be used. Because the reference standard substance 6 is thus prevented from exposure to moisture, it is long-time stable. Because the dye of the reference standard substance 6 is protein bound, the optical properties are similar to the properties of the dye when applied to a patient.

The calibration aid may be assembled as follows.

First, the standard reference substance 6 is prepared by dissolving ICG dye and albumin protein in water. Then, the carrier sheet 1 is soaked therein and dried. After drying the carrier sheet 1, a thin well defined layer of protein bound dye is present at the surface of the fleece material. The carrier sheet 1 and the backing sheet 2 are laminated into a plastic card. For this, the plastic layers 4a, 4b may be laminated tightly together in the framing region 5 for example by welding or by use of adhesive.

The plastic card is then sterilized and packed into a sealed package.

Since the calibration aid is sterile, it can be used intra-operatively. However, the calibration aid should not be placed on the tissue itself to prevent interference of the fluorescence signal emitted by the tissue with the fluorescence signal emitted by the reference standard substance 6. Typically the calibration aid should be placed near the tissue of interest (e.g. on the operating table close to the patient). In cases where it is not possible to place the calibration aid beside the patient, because the standard would be too far away from the tissue of interest, the tissue has first to be covered by a material which is opaque for near-infrared light (e.g. an opaque sterile drape or abdominal pad) and the calibration aid has then to be placed on this opaque material.

The calibration aid is intended for single use during a fluorescence measurement (duration of the measurement typically about 8 minutes) and has to be discarded after the measurement.

## Claims

1. Calibration aid for fluorescence measurement applications,
said calibration aid comprising
- a porous carrier body (1) having a front side and a backside
- a dry reference standard substance (6) adhering to at least part of the surface of said carrier body, said reference standard substance (6) comprising a fluorescent dye.

2. Calibration aid as claimed in claim 1, wherein said fluorescent dye comprises indocyanine green.

3. Calibration aid as claimed in any of the preceding claims, wherein said reference standard substance (6) further comprices albumin protein.

4. Calibration aid as claimed in any of the preceding claims, wherein said porous carrier body (1) is a textile carrier sheet (1).

5. Calibration aid as claimed in claim 4, wherein said textile carrier sheet (1) comprises a fleece material.

6. Calibration aid as claimed in any of claims 4-5, wherein said textile carrier sheet comprises polyvinyl alcohol fibers.

7. Calibration aid as claimed in any of the preceding claims, further comprising a backing sheet (2) at least partially covering the backside of said carrier body (1).

8. Calibration aid as claimed in claim 7, wherein said backing sheet (2) is arranged such that it laterally extends beyond said carrier body in at least one direction.

9. Calibration aid as claimed in any of claims 7-8, wherein said backing sheet (2) has indicia (3) printed thereon.

10. Calibration aid as claimed in any of claims 7-9, wherein said backing sheet (2) includes paper material.

11. Calibration aid as claimed in any of the preceding claims, further comprising at least one transparent plastic layer (4a, 4b) configured to serve as a humidity barrier for protection of the carrier body (1) against humidity.

12. Calibration aid as claimed in claim 11, wherein the plastic layer (4a, 4b) includes PVC film material.

13. Calibration aid as claimed in any of the previous claims, wherein the frontside of said carrier body (1) extends over an area of between 100 an 5000 square millimeters.

14. Calibration aid as claimed in any of the previous claims, wherein the complete outer surface of said calibration aid is sterile.

15. Calibration kit comprising a calibration aid as claimed in claim 14 and packaging material enclosing said calibration aid and configured to keep the outer surface of said calibration aid sterile.

16. Method for producing a calibration aid for fluorescence measurement applications, comprising the steps of
- providing a porous carrier body (1) having a front side and a backside,
- dissolving a fluorescent dye in a hydrophilic solvent to form a solution,
- applying said solution onto said carrier body (1), and
- drying said carrier body (1) in order to remove said hydrophilic solvent from said carrier body (1).

17. Method according to claim 16, further comprising the step of dissolving albumin proteine in said hydrophilic solvent prior to applying said solution onto said carrier body (1).

18. Method according to any of claims 16-17 wherein said hydrophilic solvent comprises water.

19. Method according to any of claims 16-18, further comprising the step of at least partially covering the backside of said carrier body (1) with a backing sheet.

20. Method according to claim 19, wherein the backside of said carrier body (1) is covered with said backing sheet (2) such that the backing sheet (2) laterally extends beyond said carrier body (1) in at least one direction.

21. Method according to any of claims 19-20, further comprising the step of printing indicia (3) onto said backing sheet (2) prior to the step of at least partially covering the backside of said carrier body (1) with said backing sheet (2).

22. Method according to any of claims 16-21, further comprising the step of laminating together said carrier body (1) with at least two layers (4a, 4b) of a transparent plastic material to form a card like object.

23. Method according to any of claims 16-22, further comprising the step of sterilizing the outer surface of said calibration aid.
